Europäisches Patentamt

European Patent Office

Office européen des brevets

(11) Publication number: **0 266 529**
**A2**

## (12) EUROPEAN PATENT APPLICATION

(21) Application number: **87113670.1**

(22) Date of filing: **18.09.87**

(51) Int. Cl.⁴: **A61M 16/06 , A61M 16/08**

The title of the invention has been amended (Guidelines for Examination in the EPO, A-III, 7.3).

(30) Priority: **24.09.86 SE 8604044**

(43) Date of publication of application:
**11.05.88 Bulletin 88/19**

(84) Designated Contracting States:
**AT CH DE FR GB LI**

(71) Applicant: **Möllstam, Anders**
**Tunvägen 17**
**S-132 00 Saltsjö-Boo(SE)**

(72) Inventor: **Möllstam, Anders**
**Tunvägen 17**
**S-132 00 Saltsjö-Boo(SE)**

(74) Representative: **Jacobsson, Rune et al**
**JACOBSSON & BILLBERG PATENTBYRA AB**
**Box 21113**
**S-100 31 Stockholm 21(SE)**

(54) **Adaptor for oxygen masks for patients.**

(57) To a gas mask (1) is connected an inspiration and suction device consisting of an evacuation hose (6), an oxygen hose (7) and a suction hose (8). The evacuation hose (6) is connected to the oxygen mask's connecting piece (4) for open communication with the oxygen mask's interior. The oxygen hose (7) is in open communication with the oxygen mask's interior and runs from an oxygen gas supply. One end of the suction hose (8), which hose evacuates the exhaled air, is in open communication with the evacuation hose's (6) interior and located at a suitable distance from the oxygen mask's (1) connecting piece (4), while the other end is connected to an evacuation system.

Fig. 1

## DEVICE IN OXYGEN MASKS FOR PATIENTS

This invention concerns an oxygen mask for patients, particularly patients undergoing surgery under anesthesia.

During the mid-Seventies a number of reports were published concerning a relationship between miscarriages among health care personnel and leakage of anesthetic gases. Since that time, an intensive research and development effort has been made to find ways to evacuate these gases from the operating room. Two methods have been presented in particular, one involving the use of a suction tube which is placed as close to the anesthetia mask as possible while the anesthetic is being administered, and the other involving the placement of an outer cover over the anesthesia mask, wherein the leaking gas is suctioned off from the space between the mask and the cover.

Through the use of these two methods, the amount of anesthetic gas leaking into the operating room today has been reduced to less than roughly 1,000 ppm.

However, the anesthetic gas administered to a patient is absorbed by the body's tissues and, to some extent, stored. Because of this storage, the gas will continue to be exhaled for a time after the patient has awakened. Some anesthetic gas will therefore be present in the air in post-operative wards, and this is a problem which has heretofore been overlooked in research and development work concerning anesthetic gas leakage.

Another problem encountered in connection with revival from anesthesia is that the patient often require oxygen for medical reasons. Their own respiration is inadequate, and they therefore require a higher proportion of oxygen in the air they breathe. This increase in the oxygen content can be provided by an oxygen mask.

This invention has been developed in order to provide a satisfactory solution to the above-mentioned problems, using simple means.

This invention concerns a device for use in an oxygen mask for patients, said mask being designed so as to cover the patient's nose and mouth, providing a seal which is as tight as is practical, and is equipped with a connecting piece, to which is coupled an inspiration and suction device; the device according to the invention is characterized primarily in that this inspiration and suction device consists of an evacuation tube which is coupled to the connecting piece, an oxygen gas tube, which is connected at one end to the interior of the oxygen mask and whose open end is connected to an oxygen supply, and of an evacuation tube to remove the exhaled air, one end of which is in open communication with the evacuation tube's interior, at a suitable distance from the connecting piece, and the other end of which is connected to an evacuation system.

Other characteristics and advantages of a device according to this invention are presented in the patent claims to follow, and in the following description of embodiments of a device according to the invention, wherein reference is made to the following enclosed schematic drawings:

Fig. 1 shows a side-view of an oxygen mask equipped with an embodiment of an inspiration and suction device according to the invention.

Fig. 2 shows the end of the device which is connected to the oxygen mask in larger scale and in perspective.

Fig. 3 shows the other end of the device in larger scale and in perspective.

Fig. 4 shows a side-view of another embodiment of a device according to the invention.

Fig. 5 shows a side-view of yet another embodiment of a device according to the invention.

Fig. 6, lastly, presents a table of test results obtained using a device according to the invention.

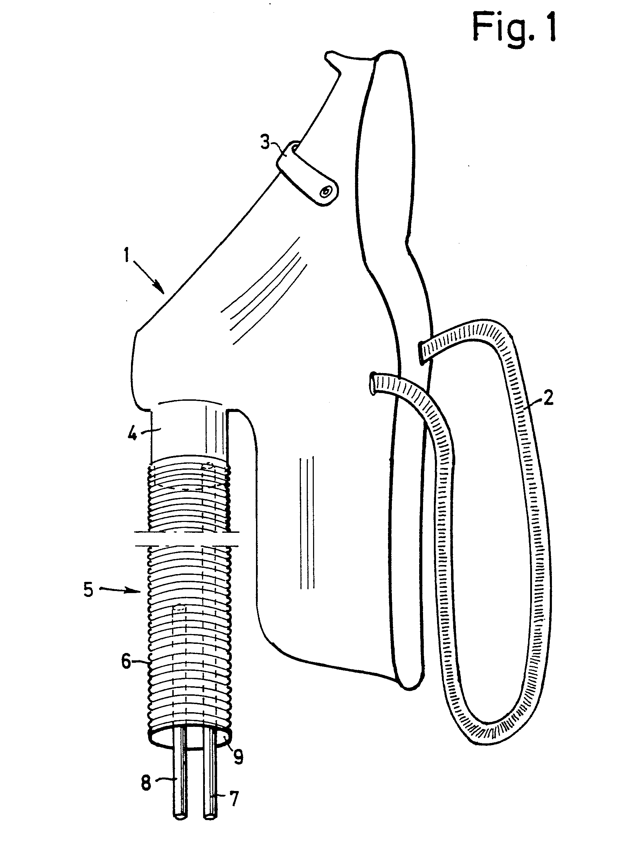

Fig. 1 shows an oxygen mask 1. It is face-shaped and preferably made of a transparent plastic material, so as not to conceal the patient's skin color. Furthermore, the mask is designed to cover the patient's nose and mouth, providing as tight a seal as is practical, and the mask's edges are rounded so as to fasten gently to the patient's face. An adjustable rubber band 2 or some similar device holds the mask in place, and a nose clamp 3 of some flexible material, such as aluminum, adapts the mask to the shape of the patient's nose. Lastly, the mask is equipped with a connecting piece 4, which is open to the patient's nose and mouth.

An inspiration and suction device, generally designated with a 5, is coupled to connecting piece 4 in a removable fashion. The primary components of this device consist of a flexible outer hose or evacuation hose 6, preferably a creased transparent plastic hose, an oxygen hose 7, preferably of plastic, and a suction hose 8, preferably of plastic as well, to remove the exhaled air, said oxygen and suction hoses being preferably of different colors.

Figs. 1-3, Fig. 4 and Fig. 5 depict examples of different embodiments of the inspiration and suction device. In all of the example embodiments, the oxygen hose 7 passes through the evacuation tube 6, with one end connected to the connecting piece 4, and the other connected to an oxygen gas supply, while one end of the suction hose is in open communication with the space between the evacuation tube and the oxygen hose, at a suitable distance from the connecting piece 4, and the other end of the suction hose is connected to an evacuation system for the exhaled air. Removal of the exhaled air occurs via the evacuation tube 6, and the aforementioned distance is important in ensuring that the exhaled air does not affect the oxygen concentration or, in other words, the aforementioned distance must be at least great enough to guarantee that the suction from the evacuation tube for each patient is not so great that it causes the oxygen concentration to fall below a value which is acceptable from a medical standpoint.

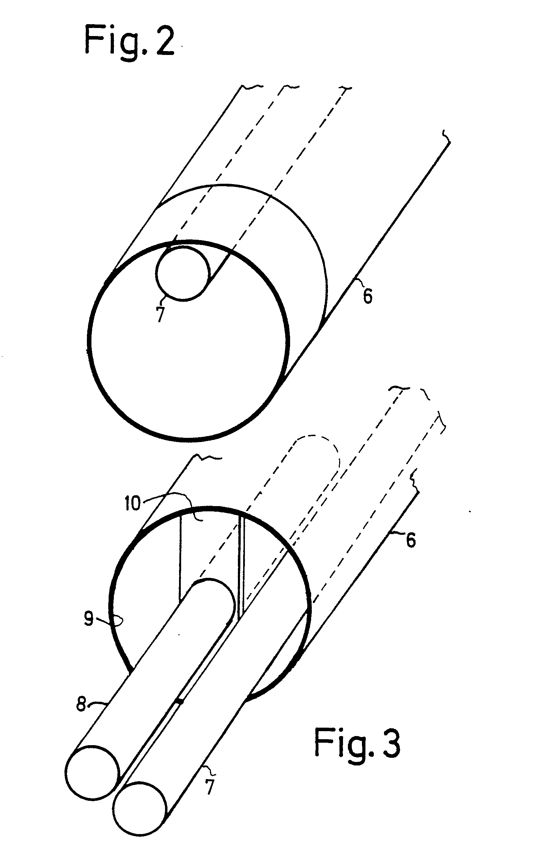

In the example embodiment shown in Figs. 1-3 both oxygen hose 7 and suction hose 8 pass through the end 9 of the evacuation hose 6 opposite connecting piece 4. As shown in Fig. 1 and 3, suction hose 8 ends inside of end 9 and is connected to this end by means of a fastening 10. This fastening 10 can be designed so as to allow for adjustment of the length of the suction hose 8. Furthermore, end 9 is open, or it can be provided with a hole. This open or hole-equipped end performs two functions, allowing the patient to continue breathing even if for some reason the flow of gas should be cut off, and preventing the occurence of excessive negative pressure in evacuation hose 6. As shown in Fig. 2, oxygen hose 7 is preferably connected to the opposite end of the evacuation hose, positioned against the evacuation hose's inner wall in order to thereby decrease the resistance to exhalation.

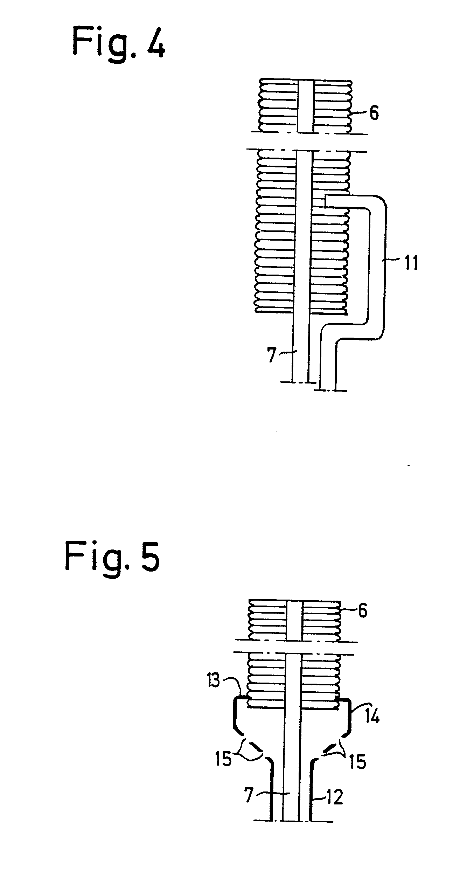

The embodiment of a device according to the invention shown in Fig. 4 differs from the embodiment presented in Figs. 1-3 in that the suction hose, designated here with the number 11, does not pass through the end of the evacuation hose, but rather runs outside of the evacuation hose to a suitable point on the evacuation hose. The sealed connection of the suction hose 11 to the evacuation hose 6 can be produced conventionally, and it is not shown in detail here. Conventional means can also be used to allow the connection to be able to adjust to the length of the evacuation hose.

Fig. 5 presents yet another embodiment of a device according to the invention. Here, as is in the previous embodiment, the oxygen hose 7 passes through the evacuation hose 6, but in this embodiment it continues on through the suction hose, designated here with the number 12. The suction hose is tightly connected at 13 to the evacuation hose 6, and is provided with an expanded section 14 below said connection, which expanded section is provided with a suitable number of safety outlet holes 15, whose function corresponds to that of the evacuation hose's open end 9 in the embodiments presented in Fig. 1-3 and 4.

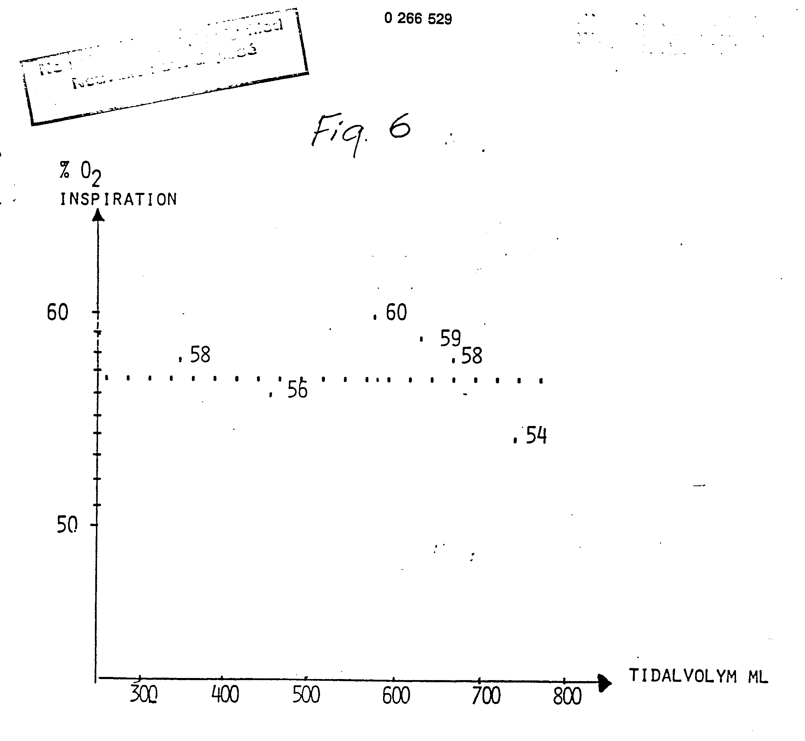

Tests have been carried out using a device according to the invention, and the test results are presented in Fig. 6. The placement of the suction hose in the evacuation hose was tested, as was the percentual oxygen content of the inspiration gas for various flows and respiration volumes. The table shows the oxygen content of the inspiration gas for various tidal volumes, in conjunction with respiration frequencies ranging from 13-19 breaths/min. The supply of oxygen gas and the suction volume remained constant throughout the testing. These tests indicate that the respiration volume influences the inspiration gas oxygen content under conditions of constant oxygen flow. An oxygen flow of 8 l/min. was used during the testing. Oxygen comprised 58% of the inspiration gas at a tidal volume of 370 ml. As the tidal volume increased, the percentual oxygen content did not alter significantly until the volume reached 700 ml/inspiration and beyond. The average percentage during the tests was 57.5%.

Naturally, the invention is not limited to the embodiments described in the foregoing text and in the appended drawings, but can be adapted in numerous ways within the framework of the patent claims to follow. The key features of an inspiration and suction device according to the invention are: an outer or evacuation hose which is open at both ends and connected to an oxygen mask, an oxygen hose which is connected to the oxygen mask to supply oxygen gas from an oxygen gas supply and a suction hose for evacuation of the exhaled air, located at a suitable distance from the point at which the evacuation hose is connected to the oxygen mask, said suction hose being in open communication with said evacuation hose. The oxygen hose preferably passes through the evacuation hose, as shown in the embodiment drawings, while the suction hose is in open communication with the space between the evacuation hose and the oxygen hose, but it is also possible for said suction hose to run from the oxygen gas supply to the oxygen mask outside of the evacuation hose.

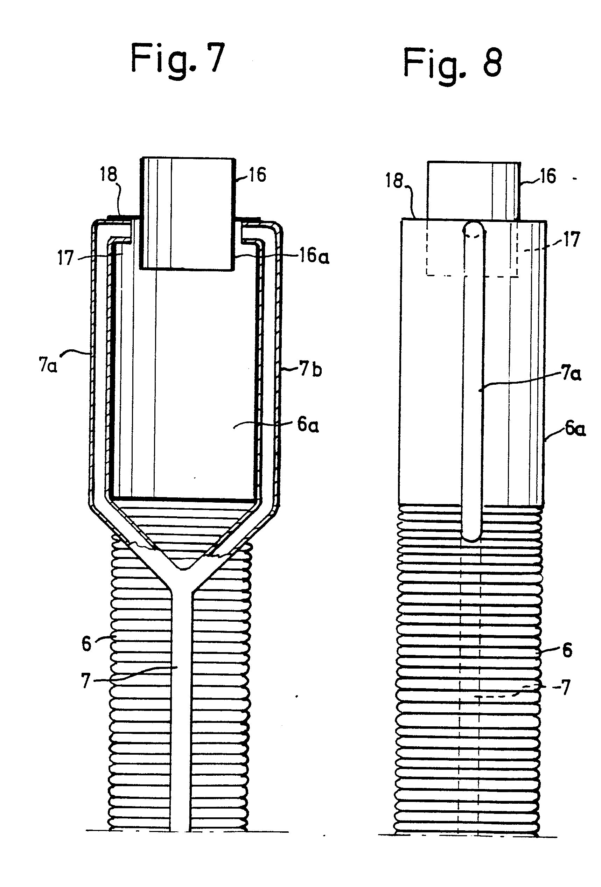

Figs. 7 and 8 show, respectively, a cross-section and a side-view in perspective of an embodiment which can generally be used in conjunction with any medical equipment employed when it is necessary to supply the patient with a specific gas at a controlled flow. More specifically, there are two primary areas of applicability for this embodiment: oxygen treatment in which an oxygen mask is connected to an oxygen gas supply, and the administering of anesthesia in which an anesthesia mask or endotracheal tube is connected to an anesthetia gas supply. In these instances, a suction hose 8 can be used, as in the previous embodiments, although such a hose is not necessary, in principle.

Figs. 7 and 8 show the closed end of the evacuation hose 6 and a connector 16 for connecting an oxygen mask, anesthesia mask, endotracheal tube or some similar equipment for the patient's respiration. These figures also depict gas hose 7, which is positioned in the center of the evacuation hose 6, and which splits into two branches 7a and 7b at a distance from the connector 16. These branches pass through the evacuation hose 6 and run along the outside of the evacuation hose, and they are in open communication with a ring-shaped channel 17 which is mounted radially outside a part 16a of the connector which is inserted into the evacuation hose 6. This channel 17 is sealed at the top by wall 18, but its opposite end is in open communication with the interior of the evacuation hose 6. Section 6a of the evacuation hose is preferably made of durable plastic, as are at least branches 7a and 7b of the gas hose.

The design of channel 17, which is oriented in the same direction as the patient's exhalations, brings about an ejector effect between the supplied gas and the exhaled gas, which occurs at the lower edge of the connector 16. When the patient inhales again, moisture and warmth will be added to the gas which is breathed in.

The essential characteristics of a device such as the one shown in Fig. 7 and 8 is that the supplied gas changes its direction of flow so that it basically coincides with the direction of flow of the patient's exhalation gases.

This can be achieved in a way other than the one shown in Figs. 7 and 8, while still remaining within the framework of patent claims 11-16 to follow. For example, the gas hose 7 can, without branching, be connected to a similar reversing channel located either inside or outside of the evacuation hose 6, or said gas hose can have more than two branches.

In prior-art rebreathing systems, the gas hose travels through either an outer cover or the evacuation hose to a connection with an oxygen or anesthesia mask, and the end of the said gas hose points towards the patient. When the patient exhales, the pressure of his exhalation must exceed the resistance from the gas flow plus the resistance of the hose material itself, including its outer cover fastener.

By using a device according to patent claims 11-16 to follow to reverse the gas flow away from the patient, thereby eliminating the resistance from this flow against the exhalation, as well as eliminating the resistance from the hose and its fastener, the exhalation process of the patient is facilitated. The aforementioned ejector effect also contributes. In addition, patients who are asleep while an anesthesia mask or an endotracheal tube is being used, as well as patients who have just awakened from anesthesia and are using oxygen masks may be affected by medication which reduces the ability to breathe, and in these cases it is particularly advantageous to facilitate the breathing process.

## Claims

1. A device for use in patient oxygen masks, wherein the oxygen mask is designed to cover the patient's nose and mouth, providing as tight a seal as is practical, said mask being equipped with a connecting piece to which is coupled an inspiration and suction device, characterized in that the inspiration and suction device consists of an evacuation hose (6) which is connected to the connecting piece (4) in open communication with the interior of the oxygen mask (1), an oxygen hose (7), which runs from an open end in communication with the oxygen mask's (1) interior to an oxygen gas supply, and a suction hose (8) for evacuating exhaled air, one end of which hose in is open communication with the interior of the evacuation hose (6) at a suitable distance from the connecting piece (4), and the other end of which is connected to an evacuation system.

2. A device according to claim 1, characterized in that the oxygen hose (7) passes through the evacuation hose (6) to the connecting piece (4), and in that the suction hose's (8) aforementioned one end is in open communication with the space between the evacuation hose (6) and the oxygen hose (7).

3. A device according to claim 1, characterized in that the oxygen hose (7) runs outside the evacuation hose (6).

4. A device according to any of claims 1-3, characterized in that the suction hose (8) runs inside of the evacuation hose (6) up to the aforementioned suitable distance.

5. A device according to any of claims 1-3, characterized in that the suction hose (11) is connected to the evacuation hose (6) from without, at the aforementioned suitable distance.

6. A device according to claim 2, characterized in that the suction hose (12) is tightly connected (13) to the end of the evacuation hose (6) opposite the connecting piece (4), and in that the oxygen hose (7) passes from the evacuation hose (6) through part of the suction hose.

7. A device according to any of the above claims, characterized in that the suction hose (8, 11, 12) is mounted in such a way as to be adjustable along the length of the evacuation hose (6).

8. A device according to any of the above claims 1-2, 4-7, characterized in that the oxygen hose (7) is mounted against the outer hose's (6) inner wall at the point where the outer hose (6) is coupled to the connecting piece (4).

9. A device according to any of the above claims, characterized by a fastener (10) for setting the length of the suction hose.

10. A device according to any of the above claims, characterized in that the end of the evacuation hose (6) opposite the connecting piece (4) is open.

11. A device for connection to a patient's respiration equipment comprising an evacuation hose for exhalation, a coupling device at the end of the evacuation hose to connect the evacuation hose and the respiration equipment via an open channel, and a gas hose which runs from a gas supply to a coupling device, characterized in that the gas hose (7, 7a, 7b) is in open communication with a channel (17) which is mounted radially outside of the coupling device's (16) channel , which channel (17) is open towards the evacuation hose (6) and closed in the other direction.

12. A device according to claim 11, characterized in that the gas hose (7, 7a, 7b) is mounted along the evacuation hose's (6) wall, at least along the extent close to the coupling device (16).

13. A device according to claim 12, characterized in that the gas hose (7, 7a, 7b) runs inside of the evacuation hose (6) up to the aforementioned extent, and outside of the evacuation hose (6) along the aforementioned extent.

14. A device according to claim 12 or 13, characterized in that the gas hose (7, 7a, 7b) divides into at least two branches (7a, 7b) along the aforementioned extent.

15. A device according to any of claims 11-14, characterized in that the channel (17) outside of the coupling device's (16) channel is formed by the inside of the evacuation hose (6) and the outside of the coupling device channel.

16. A device according to any of claims 11-15, characterized by a suction hose for evacuating the exhaled air, one end of which is in open communication with the interior of the evacuation hose (6) at a suitable distance from the coupling device (16), and the other end of which is connected to an evacuation system.

Fig. 1

**Fig. 2**

**Fig. 3**

# Fig. 4

6

11

7

# Fig. 5

6

13

14

15  15

7  12

Fig. 6

# Fig. 7

# Fig. 8